# EUROPEAN PATENT SPECIFICATION

(11) **EP 1 541 671 B1**
(45) Date of publication and mention of the grant of the patent: **05.11.2008**
(21) Application number: 02745862.9
(22) Date of filing: 05.07.2002
(51) Int. Cl.: C12N 1/18, A21D 8/04, C12P 7/52

(54) **NOVEL BAKER'S YEAST STRAINS AND BREAD USING THE SAME**
NEUE BÄCKERHEFESTÄMME UND BROT UNTER VERWENDUNG DAVON
NOUVELLE LEVURE DE BOULANGER ET PAIN FAIT AVEC CETTE DERNIÈRE

(43) Date of publication of application: 15.06.2005
(73) Proprietor: Japan Tobacco Inc., Tokyo 105-8422 (JP)
(72) Inventor: NAGASAWA, Atsushi, Japan Tobacco Inc., Tokyo 144-0042 (JP)
(74) Representative: Reinhard - Skuhra - Weise & Partner
(86) International application number: PCT/JP2002/006844
(87) International publication number: WO 2004/005490

(56) References cited:
- EP-A1- 1 036 841
- JP-A- 6 000 052
- WINZELER E A ET AL: "FUNCTIONAL CHARACTERIZATION OF THE S. CEREVISIAE GENOME BY GENE DELETION AND PARALLEL ANALYSIS" SCIENCE, AMERICAN ASSOCIATION FOR THE ADVANCEMENT OF SCIENCE, NEW YORK, US, vol. 285, no. 5429, 6 August 1999 (1999-08-06), pages 901-906, XP001025980 ISSN: 0036-8075
- EDEN A ET AL: "Two yeast homologs of ECA39, a target for c-Myc regulation, code for cytosolic and mitochondrial branched-chain amino acid aminotransferases." THE JOURNAL OF BIOLOGICAL CHEMISTRY. 23 AUG 1996, vol. 271, no. 34, 23 August 1996 (1996-08-23), pages 20242-20245, XP002112362 ISSN: 0021-9258
- MARISKA LILLY, FLORIAN F. BAUER, GUSTAV STYGER, MARIUS G. LAMBRECHTS, ISAK S. PRETORIUS: "The effect of increased branched-chain amino acid transaminase activity in yeast on the production of higher alcohols and on the flavour profiles of wine and distillates" FEMS YEAST RESEARCH, 31 March 2006 (2006-03-31), XP002389179

## Description

### FIELD OF THE INVENTION

The present disclosure relates to novel bakers' yeast strains that are freeze-tolerant and whose offensive taste and odor characteristic of yeast is very weak, as well as to bread made using the same.

### RELATED ART

In recent years, bread making techniques using frozen doughs have carried increasing weight in the bread making industry because they are advantageous, e.g., in providing fresh bread hot from the oven and in improving the efficiency of bread making processes to reduce working hours. To prepare a frozen dough, bread ingredients such as wheat flour, sugar, salt, fat, yeast and water are mixed and moulded, followed by frozen storage at around -20°C. The frozen dough thus prepared is thawed (if necessary) and subjected to final proof before being baked. When provided for long-term frozen storage, a frozen dough usually contains a 2- to 3-fold excess amount of yeast as compared to traditional bread, because even freeze-tolerant yeast strains are slightly damaged by freezing, and also to reduce the time required for final proof after thawing. To minimize the risk of freezing damage in yeast cells, a no-time dough process is also commonly employed which needs little time for fermentation after mixing. However, if the amount of yeast is increased, the offensive taste and odor characteristic of yeast will become stronger, making the flavor of the bread unpleasant. Likewise, a no-time dough which needs little time for fermentation also has a stronger offensive taste and odor characteristic of yeast because the fermentation flavor is weak in this dough, thus resulting in a more unpleasant bread flavor.

In a traditional bread making process (i.e., non-freezing process) which uses a non-frozen dough, the bread flavor is also unpleasant, for the same reason as stated above, if a short fermentation process is used to reduce the time required for the bread making process.

Moreover, in a case where conventional bakers' yeast strains are used to make breads such as those supplemented with fats (e.g., expensive cultured butter, sour cream) or those based on fermented starters (e.g., panettone starter, liquor yeast ferment), the offensive taste and odor characteristic of bakers' yeast would mask the aroma of these raw materials and impair the flavor.

JP A 6000052 describes a *Saccaromyces cerevisiae* SANK5019 isolated from sea water and bread prepared using said yeast that contains less than 112 ppm isobutyric acid and has a weaker offensive of odor than bread prepared using commercial bakers' yeast. This document does not describe that the yeast can be employed in the frozen dough method and as such, this yeast strain is not considered to be freeze-tolerant.

Winzeler E. et al: "Functional characterization of the S. cerevisiae genome by gene deletion and parallel analysis" Science, American Association for the Advancement of Science, New York, US, volume 285, No: 5429, August 6, 1999, pages 901 - 906, Eden A. et al "Two yeast homologs of ECA39, a target for C-Myc regulation, code for cytosolic and mitochondrial branched-chain amino acid aminotransferases" the Journal of Biological Chemistry, August 23, 1996, volume 271, No: 34, pages 20242 - 20245, and Mariska Lilly, Florian F. Bauer et al "The effect of increased branched-chain amino acid transaminase activity in yeast on the production of higher alcohols and on the flavour profiles of wine and distillates" FEMS Yeast Research, March 31, 2006 describe yeast strains with deletion mutation in the branched-chain amino acid transaminase BAT2/ECA40. The latter document discloses that *Saccharomyces cerevisiae* BY4742 with a deletion of BAT2 produces less than half the amount of isobutyric acid compared to the parental strain under various culturing conditions.

However, none of these documents discloses freeze-tolerant bakers' yeast suitable for producing frozen dough.

EP-A1-1 036 841 discloses super-tolerant yeast for confectionery and bakery. Said yeast does, however, not produce lower levels of isobutyric acid.

### SUMMARY OF THE INVENTION

The present disclosure provides a novel bakers' yeast strain whose offensive taste and odor characteristic of yeast is very weak. The present disclosure also provides a novel freeze-tolerant bakers' yeast strain whose offensive taste and odor characteristic of yeast is very weak. The yeast strains of the present disclosure enable the production of bread with an excellent flavor while eliminating adverse effects due to the offensive taste and odor characteristic of yeast.

### BRIEF DESCRIPTION OF DRAWINGS

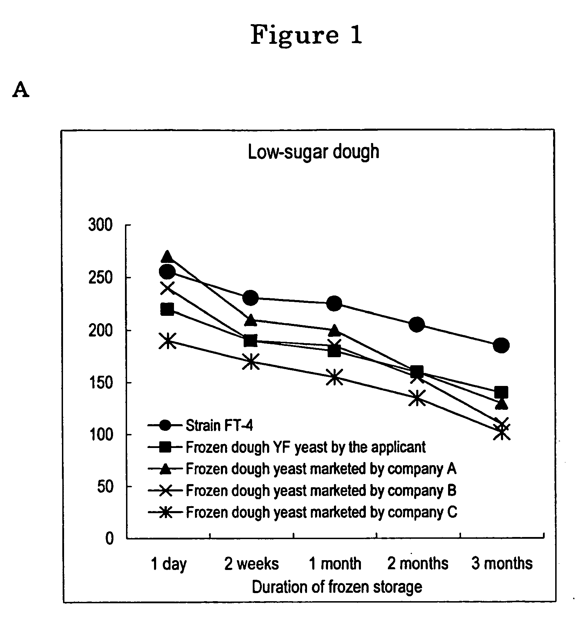

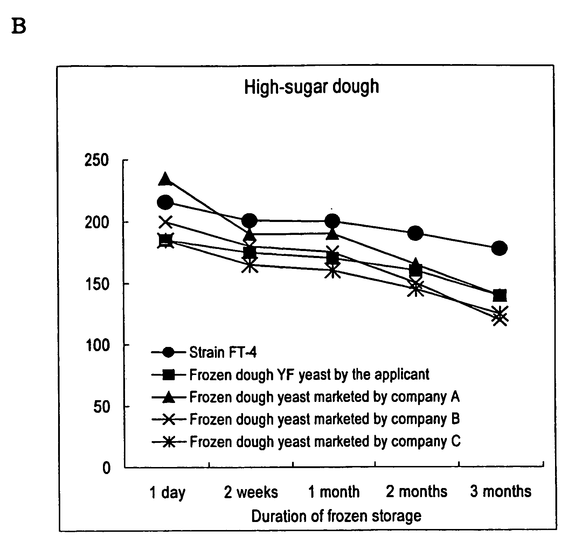

Figure 1 is a graph showing the fermentability of bread doughs prepared using the yeast strain FT-4 of the present disclosure and several comparative yeast strains, as measured by the total gas volume generated for 120 minutes. The bread doughs are frozen and stored for 1 day to 3 months and then thawed before being tested. Figure 1A shows the results obtained in low-sugar doughs, while Figure 1B shows the results obtained in high-sugar doughs.

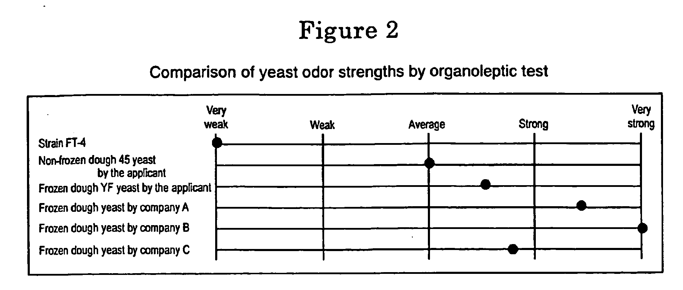

Figure 2 is a graph showing the results of an organoleptic test performed to evaluate the yeast odor of bread doughs prepared using the yeast strain FT-4 of the present disclosure and several comparative yeast strains.

### DETAILED DESCRIPTION OF THE INVENTION

To overcome the problems stated above, the inventors of the present disclosure have attempted hybridization breeding or classical mutation breeding between stored strains of bakers' yeast, liquor yeast or the like and have succeeded in finding, among the resulting strains, bakers' yeast strains that have high fermentability and whose offensive taste and odor characteristic of yeast is very weak. This finding led to the completion of the present disclosure.

Namely, a novel bakers' yeast strain *Saccharomyces cerevisiae* FT-4 found in the present disclosure has a very weak, almost undetectable, offensive taste and odor characteristic of yeast. Further, the strain FT-4 has a higher freeze tolerance and shows a fermentation capacity over a wider range of sugars when compared to the applicant's existing strains of freeze-tolerant yeast.

Moreover, the use of the novel bakers' yeast strain *Saccharomyces cerevisiae* FT-4 of the present disclosure enables the production of bread with a pleasant flavor, in which the offensive taste and odor characteristic of yeast is clearly weaker than that of bread made using traditional bakers' yeast strains.

The present invention will be further described in detail.

The term "bakers' yeast strain" as used herein is not limited to *Saccharomyces cerevisiae* and also includes *Saccharomyces rosei, Saccharomyces uvarum, Saccharomyces chevalieri* and *Torulaspora delbrueckii*. In some cases, the term can also encompass *Kluyveromyces thermotolerans* and other Saccharomyces species.

Microbiological properties of the *Saccharomyces cerevisiae* strain FT-4, a representative bakers' yeast strain obtained in the present disclosure, will be shown below. This strain was deposited with the National Institute of Advanced Industrial Science and Technology (Central 6, 1-1-1 Higashi, Tsukuba, Japan) on June 20, 2002 under Accession No. FERM BP-8081.

### Microbiological properties of the Saccharomyces cerevisiae strain FT-4

(i) Morphology: Yeast cells were cultured in YPD medium and observed under a microscope.
(ii) Size: Yeast cells were cultured in YPD medium and observed under a microscope, as in the case of morphology.
(iii) Sporulation: Yeast cells grown on YPD agar medium were inoculated onto Sharman agar medium, cultured at 20°C to 25°C for 3 to 10 days, and observed under a microscope to confirm the presence or absence of sporulation. The composition of YPD medium, YPD agar medium and Sharman agar medium are shown in Table 1.

**Table 1 Medium composition**

| | YPD medium | YPD agar medium | Sherman agar medium |
|---|---|---|---|
| Yeast extract | 5 g | 5 g | 1 g |
| Peptone | 10 g | 10 g | - |
| D-glucose | 40 g | 40 g | 0.5 g |
| KH₂PO₄ | 5 g | 5 g | - |
| MgSO₄·7H₂O | 2 g | 2 g | - |
| CH₃COOK | - | - | 1 g |
| Agar | - | 20 g | 20 g |
| Distilled water | 1000 ml | 1000 ml | 1000 ml |
| Adjusted pH | 5.5 | 5.5 | 7.2 |

(iv) Carbon source assimilation and fermentation:
Assimilation was analyzed as follows. A loopful of fresh yeast cells grown on YPD agar medium was suspended in 5 ml sterilized water, washed twice with sterilized water by centrifugation and then suspended again in 5 ml sterilized water. The suspension thus obtained (0.1 ml) was inoculated into tubes (Sarstedt tubes, 101 mm × 16.5 mm) containing 5 ml sterilized medium supplemented with various carbon sources, respectively (Yeast nitrogen base 0.67 g, various carbon sources 0.1 g each, water 10 ml), grown in shaking culture at 30°C for 48 hours and then measured for absorbance at 660 nm to determine cell growth by the degree of turbidity. To analyze fermentation, a yeast cell suspension prepared as described above (0.1 ml) was inoculated into glass tubes (180 mm × 15 mm) containing the same medium (10 ml) and equipped with a Durham's tube, grown in static culture at 30°C for 1 week and then confirmed for the presence or absence of air bubbles in the Durham's tube.
(v) Nitrate assimilation: Nitrate medium (Yeast carbon base 1.17 g, potassium nitrate 7.8 g, water 10 ml) was dispensed into Sarstedt tubes (5 ml per tube), sterilized and then inoculated with 0.1 ml of a yeast cell suspension prepared in the same manner as described for the test of carbon source assimilation. After shaking culture at 30°C for 48 hours, the tubes were measured for absorbance at 660 nm to determine cell growth by the degree of turbidity.
(vi) Vitamin requirement: Vitamin-deficient medium (Vitamin free-base 1.67 g, each vitamin solution 0.5 ml, water 10 ml) was dispensed into Sarstedt tubes (5 ml per tube), sterilized (provided that the vitamin solutions were each sterilized and cooled before being added through a sterile filter), and then inoculated with 0.1 ml of a yeast cell suspension prepared in the same manner as described for the test of carbon source assimilation. After shaking culture at 30°C for 48 hours, the tubes were measured for absorbance at 660 nm to determine cell growth by the degree of turbidity.

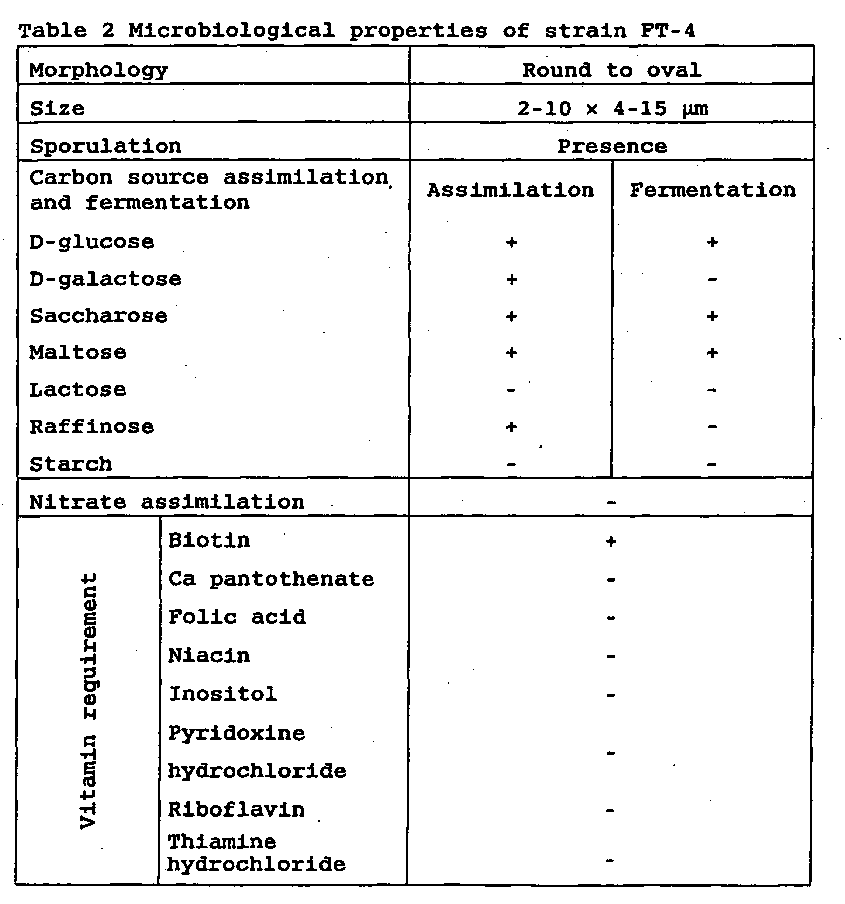

As shown in the results of Table 2, the test strain can be identified as a strain of *Saccharomyces cerevisiae.*

The term "offensive taste and odor characteristic of yeast" as used herein is intended to mean a muddy or fishy flavor which is clearly felt, particularly when fresh yeast cells are eaten directly. This flavor is also called yeast odor. Such a flavor can also be found in frozen doughs when prepared using yeast at a ratio of 7 or more relative to wheat flour which is set to 100. Likewise, bread made by a non-freezing process (which uses only 2% to 3% yeast) also has an offensive taste and odor characteristic of yeast, but such a taste and odor is accepted as a part of bread flavor in the case of using conventional yeast.

Although substances responsible for the offensive taste and odor characteristic of yeast are composed of a wide variety of compounds and are difficult to restrict to one kind of compound, it is estimated that isobutyric acid which is known as a branched fatty acid having a foul odor may be one of the causative substances. When dry cells of the *Saccharomyces cerevisiae* strain FT-4 obtained in the present disclosure, which is characterized by having a very weak offensive taste and odor characteristic of yeast, are disrupted using beads and measured for the content of isobutyric acid, it has been confirmed that traditional freeze-tolerant yeast strains have an isobutyric acid content in dry cells of 300 to 1,000 ppm, whereas the *Saccharomyces cerevisiae* strain FT-4 has an isobutyric acid content in dry cells of 125 ppm, which is less than one-half of conventional bakers' yeast strains.

The term "freeze-tolerant" or freeze tolerance" as used herein is intended to mean that a yeast strain is free from or resistant to damage caused by freezing. By way of example, a dough is prepared and allowed to ferment in a thermostat at 30°C for 60 minutes. After the completion of fermentation, the dough is divided into 30 g portions, rounded into balls and then frozen at -20°C. One day later, the dough balls are allowed to ferment while thawing at 38°C for 120 minutes and measured for the volume of carbon dioxide gas generated during fermentation, which is defined as fermentability after 1 day freezing. For comparison, the same dough is frozen for 1 month, allowed to ferment while thawing at 38°C for 120 minutes and measured for the volume of carbon dioxide gas generated during fermentation, which is defined as fermentability after 1 month freezing. Assuming that the fermentability after 1 day freezing is set to 100, the percentage of the fermentability after 1 month freezing is determined and defined as persistence of fermentability. In such a case, for example, a freeze-tolerant yeast strain will show 80% or more persistence of fermentability in a low-sugar dough (sugar content: 5%) and 90% or more persistence of fermentability in a high-sugar dough (sugar content: 25%).

A non-freezing process can be presented as an opposite example of the frozen dough process. The non-freezing process involves a series of steps, starting with dough mixing, fermentation (including primary and secondary fermentation), dividing, moulding, final proof and baking, which steps are continuously performed without freezing the dough. A wide variety of processes have been used conventionally, such as a sponge dough process, a straight dough process, a no-time dough process, soaker process and an old dough process. Although the novel bakers' yeast strains obtained in the present disclosure are freeze-tolerant, there is no problem in using them in such non-freezing processes as listed above.

The sugar content mentioned above is expressed in % by weight relative to wheat flour which is set to 100. In general, a non-sugar dough refers to a dough containing no sugar. A low-sugar dough refers to a dough containing less than 10% sugar, but a dough having a sugar content of 5% is used herein as a low-sugar dough. A high-sugar dough usually refers to a dough whose sugar content is from 20% to less than 35%, but a dough having a sugar content of 25% is used herein as a high-sugar dough.

To obtain the bakers' yeast strains of the present disclosure, which may be freeze-tolerant and are also characterized by having a very weak offensive taste and odor characteristic of yeast, sexual reproduction may be performed between a monoploid yeast strain obtained by germinating spores of a diploid bakers' yeast strain which may be freeze-tolerant and another monoploid yeast strain obtained by germinating spores of a diploid alcohol or wiled-type yeast strain whose offensive taste and odor characteristic of yeast is weak, followed by the screening procedure described in Example 1 to select, from among the resulting yeast strains, those having a weak offensive taste and odor characteristic of yeast. Further, before or after the above screening step, the screening procedure described in Example 1 may also be performed to select freeze-tolerant strains, thus providing freeze-tolerant yeast strains having a very weak offensive taste and odor characteristic of yeast. Such strains are preferred embodiments of the present disclosure. Furthermore, UV irradiation may be performed on freeze-tolerant bakers' yeast strains to cause mutations under conditions where around 99% is killed, followed by the same screening procedure to provide freeze-tolerant strains having a very weak offensive taste and odor characteristic of yeast.

### EXAMPLES

The present invention will be further described in more detail in the following examples, which are not intended to limit the scope of the invention.

### Example 1 Breeding and screening of Saccharomyces cerevisiae strain FT-4

Spores of a diploid yeast strain (the applicant's YF yeast) belonging to *Saccharomyces cerevisiae* which was at least highly freeze-tolerant were germinated to obtain a monoploid yeast strain. Likewise, spores of a diploid yeast strain (the applicant's sake yeast) belonging to *Saccharomyces cerevisiae* which was not freeze-tolerant, but whose offensive taste and odor characteristic of yeast was weak were germinated to obtain another monoploid yeast strain. These monoploid yeast strains were crossed via sexual reproduction to give a large number of diploid yeast strains.

Subsequently, primary screening for freeze tolerance was performed on these strains. YPD liquid medium (3 ml) was introduced into 13 ml tubes, sterilized at 121°C for 15 minutes and then inoculated with a loopful of each strain, followed by shaking culture at 30°C for 20 hours. The resulting yeast cells of each strain were collected by centrifugation at 3,000 rpm for 15 minutes, washed twice with cold water and then collected again. The collected yeast cells were suspended in 3 ml LF medium (prepared by dissolving 10 g glucose, 30 g maltose and 30 g sucrose in water to 1 L). After static fermentation at 30°C for 1 hour, centrifugation was performed at 3,000 rpm for 10 minutes and the resulting supernatant was measured for its ethanol content using a biosensor BF-4 (Oji Scientific Instruments). The cells were suspended again by vortexing and then frozen and stored at -20°C for 2 weeks. After 2 weeks, fermentation was effected at 30°C for 1 hour while thawing the cells. Centrifugation was performed at 3,000 rpm for 10 minutes and the resulting supernatant was measured for its ethanol content using a biosensor BF-4 (Oji Scientific Instruments). The amount of ethanol produced before freezing was subtracted from the value measured for each strain to calculate the amount of ethanol produced after thawing, followed by selecting strains which produced ethanol in an amount constituting 40% or more of the final ethanol concentration.

Likewise, secondary screening for freeze tolerance was performed as follows. YPD liquid medium (100 ml) was dispensed into 500 ml baffled Erlenmeyer flasks, sterilized at 121°C for 15 minutes and then inoculated with a loopful of each strain, followed by shaking culture at 30°C for 20 hours. The cultured solutions were transferred to centrifuge tubes and centrifuged at 3000 rpm for 15 minutes to collect the cells, followed by washing twice with cold water to give the yeast cells.

The resulting strains were then measured for their fermentability in low-sugar doughs. Individual raw materials of a low-sugar dough listed in Table 5 were charged into a gram mixer to prepare a dough for each strain under the following conditions: a mixing time of 2 minutes and a final mixed dough temperature of 30°C. The resulting doughs were each allowed to ferment in a thermostat at 30°C for 60 minutes. After the completion of fermentation, each dough was divided into 30 g portions, rounded into balls and then frozen at -20°C. One day later, the dough balls were allowed to ferment while thawing at 38°C for 120 minutes and measured with a fermograph (ATTO Corporation) for the volume of carbon dioxide gas generated during fermentation, which was defined as fermentability after 1 day freezing. For comparison, the same doughs were each frozen for 1 month, allowed to ferment while thawing at 38°C for 120 minutes and also measured with a fermograph for the volume of carbon dioxide gas generated during fermentation, which was defined as fermentability after 1 month freezing. Assuming that the fermentability after 1 day freezing was set to 100, the percentage of the fermentability after 1 month freezing was determined as persistence of fermentability to select strains showing 80% or more persistence of fermentability.

The resulting freeze-tolerant strains were screened for the offensive taste and odor characteristic of yeast. Primary screening was performed as follows. As in the case of the test described above, each strain was grown in a 500 ml baffled Erlenmeyer flask, collected and washed to give yeast cells. These yeast cells were subjected to an organoleptic test by 10 subjects to select strains having a weak offensive taste and odor characteristic of yeast.

Secondary screening was performed as follows. After being adjusted with distilled water to a solid content of 20%, the yeast cells were disrupted using beads and measured for isobutyric acid content in the supernatant by gas chromatography (Shimadzu Corporation), followed by selecting yeast cells having an isobutyric acid content of 200 ppm or less on a dry cell basis.

Four strains were selected which satisfied the above requirements and produced successful results in a bread making test. One of them was designated *Saccharomyces cerevisiae* strain FT-4.

### Example 2 Comparison of isobutyric acid content

A loopful of the strain FT-4 or bakers' yeast strains marketed by various companies was inoculated into a 500 ml baffled Erlenmeyer flask containing 100 ml YPD liquid medium and then grown in shaking culture at 30°C for 20 hours. The cultured solutions were each centrifuged at 3,000 rpm for 5 minutes to collect the cells, followed by washing twice with sterilized distilled water. The resulting washed yeast cells were frozen and stored. The freeze-dried yeast cells were reconstituted with cold water and adjusted to a solid content of 20%, followed by addition of 10% glass beads to disrupt the yeast cells by vortexing at high speed. After centrifugation at 10,000 rpm for 10 minutes, the supernatant was measured for its isobutyric acid content by gas chromatography to determine an isobutyric acid content calculated on a dry cell basis for each strain.

Table 3 shows the measured results. The strain of the present disclosure had an isobutyric acid content less than one-half of the comparative bakers' yeast strains.

**Table 3 Comparison of isobutyric acid content among bakers' yeast strains marketed by various companies**

| | Isobutyric acid content |
|---|---|
| Strain FT-4 | 125 ppm |
| Non-frozen dough 45 yeast by the applicant | 375 ppm |
| Frozen dough YF yeast by the applicant | 310 ppm |
| Frozen dough yeast 1 marketed by Company A | 530 ppm |
| Frozen dough yeast 2 marketed by Company A | 360 ppm |
| Frozen dough yeast 1 marketed by Company B | 595 ppm |
| Frozen dough yeast 2 marketed by Company B | 990 ppm |
| Frozen dough yeast marketed by Company C | 395 ppm |
| Frozen dough yeast marketed by Company D | 730 ppm |

### Example 3 Fermentability in non-frozen doughs

In accordance with the following steps, cells of the strain FT-4 were cultured in a 300 L jar fermentor and used for measurement of the fermentability in non-frozen doughs using the method of the Japan Yeast Industry Association.

### Culture of the strain FT-4

### (1) Culture of seed yeast

A loopful of the strain FT-4 was seeded into a 500 ml baffled Erlenmeyer flask containing 200 ml YPD liquid medium and then grown in shaking culture at 30°C for 24 hours. The cultured solution was then inoculated into a 30 L jar fermentor containing 15 L molasses medium (see Table 4) and grown under the conditions shown in Table 4 to give seed yeast for 300 L jar fermentor culture.

### (2) 300 L jar fermentor culture

The main culture medium shown in Table 4 was prepared in a volume of 150 L in a 300 L jar fermentor and inoculated with the whole volume of the seed culture grown in a 30 L jar fermentor, followed by culture under the following conditions.

**Table 4 Culture conditions for strain FT-4**

| Ingredient composition | Seed culture | Main culture | Remarks |
|---|---|---|---|
| Prepared sugar solution | 3.5 L | 30 L | Waste molasses from -Philippine, adjusted to a sugar concentration of 40% |
| Urea | 35 g | 800 g | |
| Monobasic ammonium phosphate | 30 g | 350 g | |
| Ammonium sulfate | 30 g | | |
| Water | 15 L | 150 L | |
| Culture conditions | | | |
| temperature | 30-32°C | 30-32°C | |
| Aeration volume | 30 L/min | 300 L/min | |
| Agitation | 400 rpm | 450 rpm | |
| PH | 5.0-6.0 | 5.0-6.0 | |
| Culture period | 16 hours | 16 hours | |

Immediately after the completion of culture, the cultured solution was centrifuged to separate yeast cells, followed by compression and dehydration using a filter fabric to give yeast cells with a water content of 65% to 67%.

### Fermentability measurement in non-frozen doughs

The fermentability under non-frozen conditions was measured for each dough composition shown in Table 5. Namely, raw materials were mixed in a gram mixer (National, USA) for 2 minutes and the resulting doughs were each divided into 30 g portions and measured for the total gas volume generated at 30°C for 120 minutes using a fermograph (ATTO Corporation).

**Table 5 Dough composition according to the method of the Japan Yeast Industry Association**

| | Wheat flour | Sugar | Salt | Yeast | Water |
|---|---|---|---|---|---|
| Non-sugar dough | 100 g | 0 g | 2 g | 2 g | 65 g |
| Low-sugar dough | 100 g | 5 g | 2 g | 2 g | 62 g |
| High-sugar dough | 100 g | 30 g | 0.5 g | 3 g | 52 g |

Table 6 shows the results obtained. When compared to the applicant's existing strains or commercially available yeasts, the strain obtained in the present invention showed high fermentability in the non-sugar and high-sugar doughs under non-frozen conditions, although it was slightly lower in the low-sugar dough, thus suggesting that this strain had broad utility over a wide range of sugars.

**Table 6 Comparison of fermentability in non-frozen doughs**

| | Strain FT-4 | Non-frozen dough 45 yeast by the applicant | Frozen dough YF yeast by the applicant | Non-frozen dough yeast marketed by Company A | Non-frozen dough yeast marketed by Company B | Non-frozen dough yeast marketed by Company C |
|---|---|---|---|---|---|---|
| Non-sugar dough | 82.8 | 72.8 | 65.2 | 68.1 | 64.6 | 70.5 |
| Low-sugar dough | 92.4 | 100.7 | 81.0 | 99.3 | 92.3 | 95.9 |
| High-sugar dough | 83.6 | 68.5 | 65.1 | 60.2 | 70.1 | 68.2 |

### Example 4 Comparison of fermentability in frozen doughs

The yeast cells obtained in Example 3 were measured for freeze tolerance in low-sugar and high-sugar doughs. In accordance with the dough composition shown in Table 7, raw materials were prepared and mixed in a gram mixer (National, USA) for 2 minutes and the resulting doughs were each allowed to ferment in an incubator at 25°C for 60 minutes. After the completion of fermentation, each dough was divided in to 30 g portions, transferred to a plastic bag, and then frozen and stored at -20°C. At 1 day, 2 weeks, 1 month, 2 months and 3 months after freezing, the doughs were removed from the freezer for the purpose of thawing, transferred to a glass bottle for fermograph measurement and measured for the total gas volume generated at 38°C for 120 minutes using a fermograph (ATTO Corporation).

**Table 7 Dough composition for frozen dough test**

| | Wheat flour | Sugar | Salt | Yeast | Water |
|---|---|---|---|---|---|
| Low-sugar dough | 100 g | 5 g | 1.5 g | 7 g | 62 g |
| High-sugar dough | 100 g | 25 g | 1.5 g | 6 g | 55 g |

Figure 1 shows a graph of the time course of the total gas volume generated for 120 minutes, while Table 8 shows the persistence of fermentability after frozen storage for 1 month. The strain FT-4 of the present disclosure showed 80% or more persistence of fermentability in the low-sugar dough and 90% or more persistence of fermentability in the high-sugar dough. As shown in the time course of fermentability over 3 months, it was also confirmed that the strain FT-4 had a satisfactory freeze tolerance capacity when compared to conventional strains.

**Table 8 Persistence of fermentability after frozen storage for 1 month**

| | Strain FT-4 | Frozen dough YF yeast by the applicant | Frozen dough yeast marketed by Company A | Frozen dough yeast marketed by Company B | Frozen dough yeast marketed by Company C |
|---|---|---|---|---|---|
| Low-sugar dough | 88.1% | 81.8% | 74.1% | 77.1% | 81.6% |
| High-sugar dough | 92.6% | 91.9% | 80.9% | 87.5% | 86.5% |

### Example 5 Comparison of yeast odor

To examine the yeast cells obtained in Example 3 for the strength of offensive taste and odor characteristic of yeast, an organoleptic test was performed by 10 subjects using the applicant's non-frozen dough 45 yeast as a reference standard. In the organoleptic test, test strains were evaluated for their yeast odor against the 45 yeast on a five-point scale (1 = very weak, 2 = weak, 3 = average (equal level), 4 = strong, 5 = very strong) to calculate a mean score for each strain.

Figure 2 shows the results obtained. When compared to other yeast strains, the strain of the present disclosure had a very weak offensive taste and odor characteristic of yeast and was almost tasteless and odorless.

### Example 6 Bread making test

In accordance with the composition and steps shown in Table 9, a bread making test was performed on the yeast cells obtained in Example 3 by preparing frozen doughs for white bread, table rolls and sweet buns. The frozen doughs were thawed at 1, 2 and 3 months and measured for the time required for final proof and their bread volume after baking.

The measurement of final proof time and bread volume and the evaluation of flavor were accomplished as follows.

Final proof time: the time required for each dough to reach its pre-determined volume (In the case of white bread doughs, the time was measured at the top of the case. In the case of table rolls and sweet buns, 100 g rounded doughs were frozen and stored separately and thawed in beakers for measurement).

Bread volume: After reaching the pre-determined volume during final proof, each dough was baked and immediately measured for its volume by the rapeseed method (n = 5 or more).

Flavor: To determine whether baked bread had a pleasant flavor, each bread sample was evaluated for its flavor by 10 subjects on a five-point scale (5 = very pleasant, 4 = pleasant, 3 = neutral, 2 = unpleasant, 1 = very unpleasant). The scores given by the 10 subjects were averaged for each bread sample and the resulting average scores were expressed as follows: "Ⓞ" = >4 points, "○" = 4 to 3 points, "Δ" = 3 to 2 points, "×" = <2 points.

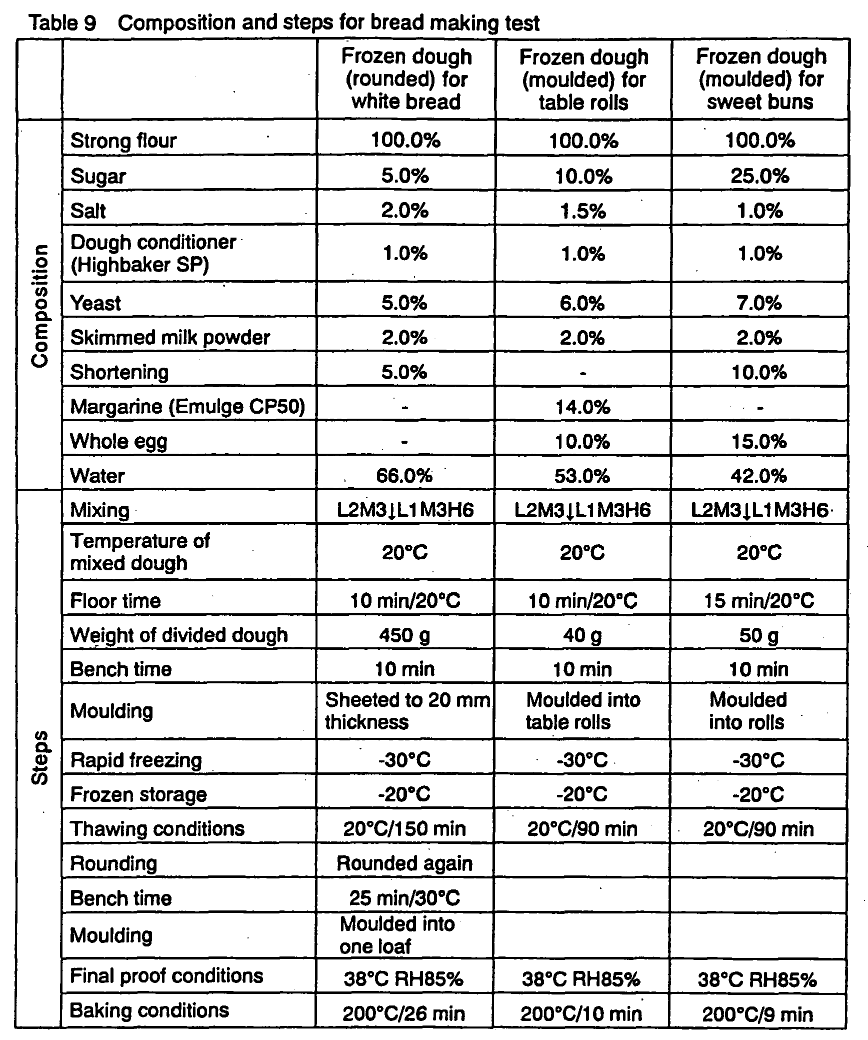

Table 10 shows the results of the bread making test. All the frozen doughs prepared using the strain of the present disclosure required less time for final proof than the existing strains; the fermentability of this strain exceeded that of the existing strains in all low-, medium- and high-sugar doughs. Moreover, the flavor of the resulting bread samples was clear and pleasant because yeast odor was weak at the stage after frozen storage for 1 month. Even after long-term frozen storage for 2 and 3 months, the flavor still remained very pleasant, not only because yeast odor was weak, but also because the smell of dead yeast cells caused by freezing damage was significantly prevented when compared to other yeast strains.

**Table 10 Results of bread making test**

| White bread | | | | | |
|---|---|---|---|---|---|
| | Duration of frozen storage | Strain FT-4 | Frozen dough YF yeast by the applicant | Frozen dough yeast marketed by Company A | Frozen dough yeast marketed by Company B |
| Final proof time | 1 month | 48 min | 47 min | 52 min | 52 min |
| | 2 months | 55 min | 58 min | 59 min | 60 min |
| | 3 months | 57 min | 62 min | 60 min | 65 min |
| Bread volume | 1 month | 2480 ml | 2340 ml | 2490 ml | 2490 ml |
| | 2 months | 2410 ml | 2345 ml | 2320 ml | 2320 ml |
| | 3 months | 2390 ml | 2350 ml | 2310 ml | 2310 ml |
| Flavor | 1 month | Ⓞ | ○ | ○ | ○ |
| | 2 months | Ⓞ | Δ | × | × |
| | 3 months | Ⓞ | × | × | × |

| Table rolls | | | | | |
|---|---|---|---|---|---|
| | Duration of frozen storage | Strain FT-4 | Frozen dough YF yeast by the applicant | Frozen dough yeast marketed by Company A | Frozen dough yeast marketed by Company B |
| Final proof time | 1 month | 34 min | 38 min | 36 min | 37 min |
| | 2 months | 38 min | 44 min | 44 min | 45 min |
| | 3 months | 39 min | 42 min | 46 min | 49 min |
| Bread volume | 1 month | 315 ml | 300 ml | 283 ml | 300 ml |
| | 2 months | 287 ml | 270 ml | 253 ml | 265 ml |
| | 3 months | 283 ml | 260 ml | 230 ml | 250 ml |
| Flavor | 1 month | Ⓞ | ○ | ○ | ○ |
| | 2 months | Ⓞ | ○ | Δ | Δ |
| | 3 months | Ⓞ | Δ | × | × |

| Sweet buns | | | | | |
|---|---|---|---|---|---|
| | Duration of frozen storage | Strain FT-4 | Frozen dough YF yeast by the applicant | Frozen dough yeast marketed by Company A | Frozen dough yeast marketed by Company B |
| Final proof time | 1 month | 42 min | 49 min | 52 min | 49 min |
| | 2 months | 55 min | 58 min | 59 min | 60 min |
| | 3 months | 56 min | 62 min | 65 min | 66 min |
| Bread volume | 1 month | 335 ml | 327 ml | 330 ml | 315 ml |
| | 2 months | 320 ml | 303 ml | 300 ml | 305 ml |
| | 3 months | 315 ml | 305 ml | 290 ml | 295 ml |
| Flavor | 1 month | Ⓞ | ○ | ○ | ○ |
| | 2 months | Ⓞ | ○ | ○ | ○ |
| | 3 months | Ⓞ | ○ | Δ | Δ |

### ADVANTAGE OF THE INVENTION

The completion of the present disclosure enables the provision of novel bakers' yeast strains that are freeze-tolerant and whose offensive taste and odor characteristic of yeast is very weak, thus enabling the production of bread with an excellent flavor while eliminating adverse effects due to the offensive taste and odor characteristic of yeast.

## Claims

1. A bakers' yeast strain deposited under accession number FERM BP-8081 belonging to Saccharomyces cerevisiae **characterized in that**:
i) said strain is sufficient freeze-tolerant to be used in the preparation of a frozen dough for bread,
ii) said strain has an isobutyric acid content in dry cells of 150 ppm or less determined for a specimen of the strain propagated under shaking in YPD liquid medium for 20 hours at 30°C,
iii) said strain is capable of fermenting a bread dough over a dough sugar content range of between 0 and less than 35 wt% relative to the flour,
wherein said strain is obtained by crossing an alcohol yeast strain and a freeze-tolerant bakers's yeast strain, and screening from among yeasts of the subsequent generation to find a strain satisfying the above requirements i) - iii), or a mutant or progeny of said strain which satisfies the above requirements i) - iii).

2. A frozen bread dough which comprises the bakers' yeast strain according to claim 1.

3. A method for preparing a frozen bread dough **characterized by** admixing yeast of the strain as claimed in claim 1 with wheat flour and other dough ingredients to provide a dough, and freezing the dough.

4. Use of the bakers's yeast of the strain as claimed in claim 1 for producing a frozen dough.

## Patentansprüche

1. Ein Bäckerhefestamm, der unter der Zugriffsnummer FERM BP-8081 hinterlegt ist und Saccharomyces cerevisiae angehört, **dadurch gekennzeichnet, dass**:
i) der Stamm ausreichend gefriertolerant ist, um bei der Herstellung eines gefroren Teigs für Brot verwendet zu werden,
ii) der Stamm einen Isobuttersäuregehalt in trockenen Zellen von 150 ppm oder weniger aufweist, bestimmt für eine Probe des Stamms, welcher unter Schütteln in YPD-Flüssigmedium für 20 Stunden bei 30 °C vermehrt wurde,
iii) der Stamm in der Lage ist, einen Brotteig über einen Teig-Zuckergehaltsbereich zwischen 0 und weniger als 35 Gew.-%, relativ zu dem Mehl, zu fermentieren,
wobei der Stamm erhalten wird durch Kreuzen eines Alkohol-Hefestamms und eines gefriertoleranten Bäckerhefestamms und Screening unter den Hefen der nachfolgenden Generation, um einen Stamm, der die obigen Anforderungen i) - iii) erfüllt, oder eine Mutante oder einen Nachkommen des Stammes, der die obigen Anforderungen i) - iii) erfüllt, zu finden.

2. Ein gefrorener Brotteig, der den Bäckerhefestamm nach Anspruch 1 einschließt.

3. Ein Verfahren zur Herstellung eines gefrorenen Brotteigs, **gekennzeichnet durch** Vermischen von Hefe des Stamms nach Anspruch 1 mit Weizenmehl und weiteren Teigzutaten, um einen Teig bereitzustellen, und Einfrieren des Teigs.

4. Verwendung der Bäckerhefe des Stamms nach Anspruch 1 zur Herstellung eines gefrorenen Brotteigs.

## Revendications

1. Souche de levure de boulanger déposée sous le numéro d'entrée FERM BP-8081 appartenant aux Saccharomyces cerevisiae, **caractérisée en ce que** :
i) ladite souche est suffisamment tolérante à la congélation pour être utilisée dans la préparation d'une pâte congelée pour du pain,
ii) ladite souche a une teneur en acide isobutyrique dans les cellules sèches de 150 ppm ou moins, déterminée pour un échantillon de la souche propagée par secouage dans un milieu liquide YPD pendant 20 heures à 30 °C,
iii) ladite souche est susceptible de fermenter une pâte à pain dans une plage de teneur en sucre de la pâte comprise entre 0 et moins de 35 % en poids par rapport à la farine,
dans laquelle ladite souche est obtenue en croisant une souche de levure alcoolique et une souche de levure de boulanger tolérante à la congélation, et en examinant les levures de la génération suivante pour trouver une souche satisfaisant aux exigences précédentes i) à iii), ou un mutant ou une descendance de ladite souche qui satisfait aux exigences précédentes i) à iii).

2. Pâte à pain congelée qui comprend la souche de levure de boulanger selon la revendication 1.

3. Procédé pour préparer une pâte à pain congelée, **caractérisé par** le mélange de la levure de la souche selon la revendication 1 avec une farine de blé et d'autres ingrédients de pâte pour réaliser une pâte, et la congélation de la pâte.

4. Utilisation de la levure de boulanger de la souche selon la revendication 1 pour produire une pâte congelée.
